(19) Europäisches Patentamt
European Patent Office
Office européen des brevets

(11) Publication number: 0 007 707 A1

(12) EUROPEAN PATENT APPLICATION

(21) Application number: 79301247.7

(22) Date of filing: 28.06.79

(51) Int. Cl.³: C 07 D 249/08
C 07 D 233/64, A 01 N 43/50
A 01 N 43/64

(30) Priority: 27.07.78 GB 3131978

(43) Date of publication of application: 06.02.80 Bulletin 80/3

(84) Designated Contracting States: BE CH DE FR GB IT NL

(71) Applicant: IMPERIAL CHEMICAL INDUSTRIES LIMITED
Imperial Chemical House Millbank
London SW1P 3JF(GB)

(72) Inventor: Worthington, Paul Anthony
22 Boulters Gardens
Maidenhead, Berkshire(GB)

(72) Inventor: Rathmell, William George
10 Gypsy Lane
Wokingham, Berkshire(GB)

(72) Inventor: Skidmore, Anthony Michael
7 Tanglewood Off Foxcote
Finchampstead Wokingham, Berkshire(GB)

(74) Representative: Alner, Henry Giveen Hamilton et al,
Imperial Chemical Industries Legal Department: Patents
Thames House North Millbank
London SWIP 4QG(GB)

(54) **1,3-Diazol- and 1,2,4-triazol-derivatives, processes for their preparation, pesticidal compositions containing them and methods of combating pests.**

(57) Compounds of the formula

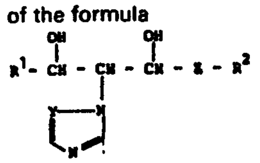

(I)

wherein $R^1$ and $R^2$ are alkyl, cycloalkyl or phenyl, Y is =N- or =CH- and z is carbonyl or a derivative thereof; and salts, metal complexes, ethers and esters thereof; and isomeres of any of the foregoing, have fungicidal activity. The compounds of the formula (I) are prepared by reacting a compound of the formula

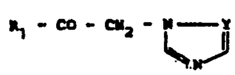

(III)

with a compound of the formula

HOC - CO - $R^2$ (IV)

to give the diketo-alcohol of the formula

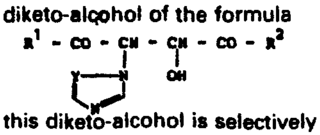

(II)

whereafter this diketo-alcohol is selectively reduced.

EP 0 007 707 A1

Croydon Printing Company Ltd.

TITLE MODIFIED see front page

HETEROCYCLIC COMPOUNDS, PROCESSES FOR THEIR PREPARATION, PESTICIDAL COMPOSITIONS CONTAINING THEM AND METHODS OF COMBATING PESTS

This invention relates to imidazole and triazole compounds useful as fungicides and plant growth regulators, to a process for preparing them, to compositions containing them, and to a method of combating fungal infections in, and regulating the growth of, plants using them.

The present invention provides compounds having the general formula (I)

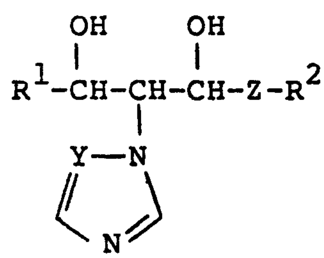

(I)

wherein each of $R^1$ and $R^2$, which may be the same or different, is unsubstituted or alkyl-substituted cycloalkyl, unsubstituted or halo-substituted alkyl or unsubstituted or substituted phenyl, Y is =N- or =CH- and Z is C = O or a derivative thereof (e.g. an imine, oxime, ketal, hydrazone or semicarbazone); and esters, ethers, acid addition salts and metal complexes thereof; and isomers of any of the foregoing.

The compounds of the invention contain chiral centres. The compounds are generally obtained in the form of racemic mixtures. However, these and other mixtures can be separated into the individual isomers by methods known in the art e.g. chromatography. In many cases, the compounds can be prepared stereospecifically in the form of a single diastereoisomer.

The alkyl groups, which can be straight or branched chain, preferably have 1 to 5 carbon atoms; examples

are methyl, ethyl, propyl (n- or i-propyl) and butyl (n-, i- or t-butyl).

Suitable substituents for the phenyl group are halogen, $C_{1-4}$ alkyl [e.g. methyl, ethyl, propyl (n- or i-propyl) and butyl (n-, i- or t-butyl)], halo($C_{1-4}$ alkyl) (e.g. chloro- or bromo- methyl), hydroxy ($C_{1-4}$ alkyl) (e.g. hydroxymethyl), phenyl, halophenyl (e.g. chlorophenyl), cycloalkyl, nitro, cyano, $C_{1-4}$ alkoxy (e.g. methoxy, ethoxy, propoxy or butoxy), $C_{2-4}$ alkenyloxy (e.g. allyloxy), ($C_{1-4}$ alkylene)dioxy (e.g. methylenedioxy), ($C_{1-4}$ alkoxy) ($C_{1-4}$ alkyl) [e.g. methoxy- or ethoxy- methyl or 2-methoxy- or ethoxy-ethyl], mercapto, ($C_{1-4}$ alkyl) thio [e.g. methyl- or ethyl-thio], ($C_{1-4}$ alkyl) sulphonyl [e.g. methyl- or ethyl-sulphonyl], ($C_{1-4}$ haloalkyl)sulphonyl [e.g. trifluoromethylsulphonyl], phenylsulphonyl, unsubstituted or mono- or di- ($C_{1-4}$ alkyl) substituted sulphamoyl or carbamoyl, 1-pyrrolidinylsulphonyl, carboxy, ($C_{1-4}$ alkoxy)carbonyl [e.g. methoxy- or ethoxy-carbonyl], hydroxy, $C_{1-6}$ alkanoyloxy, benzoyloxy, carboxy ($C_{1-4}$ alkyl)oxy (e.g. carboxymethoxy or 1-carboxyethoxy), unsubstituted or mono- or di- ($C_{1-4}$ alkyl) substituted amino, ($C_{1-6}$ alkanoyl)amino, N-($C_{1-4}$ alkyl)-N-($C_{1-6}$ alkanoyl)amino, formylamino, N-($C_{1-4}$ alkyl) formylamino, phenylethyl, methylenedioxyphenyl, phenoxy or benzyloxy. A suitable alkanoyl is acetyl or propionyl. The phenyl group can have more than one substituent; examples of polysubstituted groups are those substituted with up to the maximum possible number (especially 1, 2 or 3) of for example halogen (particularly chlorine) atoms and/or nitro, methyl or methoxy groups.

Examples of suitable phenyl groups are phenyl itself, chlorophenyl (for example o-, m- or p-chlorophenyl), dichlorophenyl (e.g. 3,4-, 2,4-, 3,5- or 2,6-dichlorophenyl), trichlorophenyl (e.g. 2,3,6- or 2,4,5-trichlorophenyl), tetrachlorophenyl, pentachlorophenyl, bromophenyl (e.g. o-, m- or p-bromophenyl), dibromophenyl

(e.g. 2,4-dibromophenyl), fluorophenyl (e.g. o-, m- or p-fluorophenyl), difluorophenyl (e.g. 2,4- or 3,4-difluorophenyl), pentafluorophenyl, iodophenyl (e.g. o-iodophenyl), aminophenyl (e.g. p-aminophenyl), methylphenyl (e.g. o-, m- or p-methylphenyl), dimethylphenyl (e.g. 2,6-, 2,5- or 3,4-dimethylphenyl), ethylphenyl (e.g. p-ethylphenyl), propylphenyl (e.g. p-i-propylphenyl), butylphenyl (e.g. p-t-butylphenyl), cyanophenyl (e.g. o-, m- or p-cyanophenyl), nitrophenyl (e.g. o-, m- or p-nitrophenyl), dinitrophenyl (e.g. 2,4-dinitrophenyl), cyanochlorophenyl (e.g. 3-cyano-4-chlorophenyl or 4-cyano-3-chlorophenyl), methylsulphonylphenyl (e.g. p-methylsulphonylphenyl), sulphamoylphenyl (e.g. p-sulphamoylphenyl), N,N-dimethylsulphamoylphenyl [e.g. p-(N,N-dimethylsulphamoyl)phenyl], pyrollidin-1-ylsulphonylphenyl (e.g. p-pyrollidin-1-ylsulphonylphenyl), trifluoromethylsulphonylphenyl (e.g. p-trifluoromethylsulphonylphenyl), methylthiophenyl (e.g. p-methylthiophenyl), (chloromethyl)phenyl [e.g. o-, m- or p-(chloromethyl)phenyl], (bromomethyl)phenyl [e.g. o-, m- or p-(bromomethyl)phenyl], (hydroxymethyl)phenyl [e.g. o-, m- or p-(hydroxymethyl)phenyl], (methoxymethyl)phenyl [e.g. o-, m- or p-(methoxymethyl)phenyl], carboxyphenyl (e.g. o-, m- or p-carboxyphenyl), methoxycarbonylphenyl (e.g. o-, m- or p-methoxycarbonylphenyl), N,N-dimethylcarbamoylphenyl [e.g. o-, m- or p-(N,N-dimethylcarbamoyl)phenyl], N,N-dimethylaminophenyl [e.g. o-, m- or p-(N,N-dimethylamino)phenyl], hydroxyphenyl (e.g. o-, m- or p-hydroxyphenyl), acetoxyphenyl (e.g. o-, m- or p-acetoxyphenyl), benzoyloxyphenyl (e.g. o-, m- or p-benzoyloxyphenyl), (trifluoromethyl)phenyl [e.g. o-, m- or p-(trifluoromethyl)phenyl], methoxyphenyl (e.g. o-, m- or p-methoxyphenyl), dimethoxyphenyl (e.g. 2,4-, 3,4- or 3,5-dimethoxyphenyl), ethoxyphenyl (e.g. o-, m- or p-ethoxyphenyl), propoxyphenyl (e.g. p-i-propoxyphenyl or p-n-propoxyphenyl), butoxyphenyl (e.g. o-, m- or p-i-butoxyphenyl), allyloxyphenyl (e.g. o-, m- or p-allyloxyphenyl), carboxymethoxyphenyl (e.g. o-, m- or p-carboxymethoxyphenyl), 1-carboxyethylphenyl [e.g. o-, m- or p-(1-carboxyethyl)phenyl], chloronitrophenyl (e.g. 3-nitro-4-chlorophenyl), fluoronitrophenyl (e.g. 2-nitro-4-fluorophenyl), chlorofluorophenyl (e.g. 2-fluoro-4-chlorophenyl, 2-chloro-6-fluorophenyl or 2-chloro-4-fluorophenyl), fluorobromophenyl (e.g. 2-fluoro-4-bromophenyl), methylenedioxychlorophenyl (e.g. 2-chloro-4,5-methylenedioxyphenyl), methoxychlorophenyl (e.g. 3-chloro-4-methoxyphenyl), methoxybromophenyl (e.g. 2-methoxy-5-bromophenyl or 3-bromo-4-methoxyphenyl), methoxynitrophenyl (e.g. 2-methoxy-5-nitrophenyl or 4-methoxy-3-nitrophenyl), ethoxynitrophenyl (e.g. 4-ethoxy-3-nitrophenyl), ethoxychlorophenyl (e.g. 4-ethoxy-3-chlorophenyl), ethoxybromophenyl (e.g. 4-ethoxy-3-bromo phenyl), benzyloxyphenyl (e.g. p-benzyloxyphenyl), phenylphenyl (e.g. p-phenylphenyl) or methylenedioxyphenylphenyl (e.g. 3,4-methylenedioxyphenylphenyl).

The cycloalkyl group suitably has 3 to 6 carbon atoms; preferably it is cyclopropyl, cyclopentyl, cyclohexyl or methylcyclohexyl.

Preferably the haloalkyl group contains 1 to 3 halogen atoms; examples are 2-chloroethyl, trifluoromethyl or trichloromethyl.

The halogen can be fluorine, chlorine, bromine or iodine.

Suitable salts are salts with inorganic or organic acids, e.g. hydrochloric, nitric, sulphuric, toluenesulphonic, acetic or oxalic acid. The esters are suitably alkanoates (e.g. acetates) and the ethers are suitably alkyl (e.g. methyl or ethyl), aryl (e.g. phenyl) or aralkyl (e.g. benzyl) ethers.

The metal complex is suitably one including copper, zinc, manganese or iron.

Examples of the triazole compounds of general formula (I) are given in Table I.

TABLE I

| COMPOUND NO | $R^1$ | $R^2$ | Z | MELTING POINT ($^oC$) |
|---|---|---|---|---|
| 1 | $-C(CH_3)_3$ | $-C(CH_3)_3$ | C=O | 180-182$^o$ |
| 2 | $-CH(CH_3)_2$ | $-C(CH_3)_3$ | C=O | 43-46$^o$ |
| 3 | $p-Cl-C_6H_4$ | $p-Cl-C_6H_4$ | C=O | - |
| 4 | $-CH(CH_3)_3$ | $p-Cl-C_6H_4$ | C=O | - |

The compounds (and the salts and complexes) of the invention may be made by selectively reducing a diketo-alcohol of general formula (II):

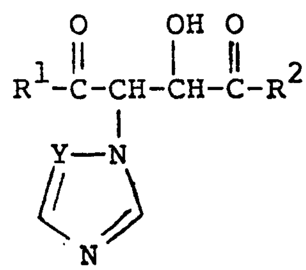

(II)

wherein Y, $R^1$ and $R^2$ are as defined above, or a salt or metal complex thereof, with for example a metal hydride reducing agent (e.g. lithium aluminium hydride, sodium borohydride or aluminium isopropoxide) in an inert polar solvent (e.g. water or ethanol).

The diketo-alcohols wherein Y is =N- are disclosed in British Patent Application No 50936/77, the disclosure of which document being incorporated herein by reference.

The diketo-alcohols of general formula (II) may be made by reacting a compound of general formula (III):

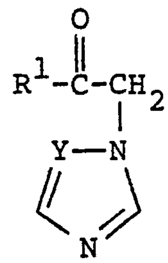

(III)

where Y and $R^1$ are as defined above, with a compound of general formula (IV):

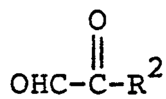

(IV)

where $R^2$ is as defined above, in the presence of a base (e.g. sodium methoxide or N-methylaniline magnesium bromide).

The compounds of general formula (III) and (IV) may be made by methods set out in the literature.

The functional derivatives, salts, metal complexes, ethers and esters of the compounds of general formula (I) can be prepared from the latter in known manner. For example, the complexes can be made by reacting the uncomplexed compound with a metal salt in a suitable solvent. The substituent on the phenyl group in the compound of general formula (I) can often be changed by methods known in the art. For example, a compound wherein for example $R^1$ is phenyl substituted with carboxy can be prepared from the corresponding compound wherein $R^1$ is phenyl substituted with alkoxycarbonyl, and *vice-versa*.

The compounds are active fungicides, particularly against the diseases:-

*Piricularia oryzae* on rice
*Puccinia recondita*, *Puccinia striiformis* and other rusts on wheat, *Puccinia hordei*, *Puccinia striiformis* and other rusts on barley, and rusts on other hosts e.g. coffee, apples, vegetables and ornamental plants

Plasmopara viticola on vines
Erysiphe graminis (powdery mildew) on barley and wheat and other powdery mildews on various hosts such as Helminthosporium spp. on cereals, Sphaerotheca fuliginea on cucurbits (e.g. cucumber), Podosphaera leucotricha on apples and Uncinula necator on vines
Cercospora arachidicola on peanuts and other Cercospora species on for example sugar beet, bananas and soya beans
Botrytis cinerea (grey mould) on tomatoes, strawberries, vines and other hosts
Phytophthora infestans (blight) on tomatoes
Venturia inaequalis (scab) on apples

Some of the compounds have also shown a broad range of activities against fungi in vitro. They have activity against various post-harvest diseases on fruit (e.g. Penicillium digatatum and italicum on oranges and Gloeosporium musarum on bananas). Further some of the compounds are active as seed dressings against: Fusarium spp., Septoria spp., Tilletia spp. (i.e. bunt, a seed borne disease of wheat), Ustilago spp., Helminthosporium spp. on cereals, Rhizoctonia solani on cotton and Corticium sasakii on rice.

The invention compounds also display plant growth regulating activity.

The compounds may be used as such for fungicidal purposes but are more conveniently formulated into compositions for such usage. The invention thus provides also a fungicidal composition comprising a compound of general formula (I) or a salt, complex, ether or ester thereof as hereinbefore defined, and a carrier or diluent.

The invention also provides a method of combating fungal diseases in a plant, which method comprises applying to the plant, to seed of the plant or to the locus of the plant or seed a compound or functional derivative, salt, metal complex, ether or ester thereof

(or an isomer of any of the foregoing) as hereinbefore defined.

The compounds, salts, complexes, ethers and esters etc., can be applied in a number of ways, for example they can be applied, formulated or unformulated, directly to the foliage of a plant, or they can be applied also to bushes and trees, to seeds or to other medium in which plants, bushes or trees are growing or are to be planted, or they can be sprayed on, dusted on or applied as a cream or paste formulation, or they can be applied as a vapour. Application can be to any part of the plant, bush or tree, for example to the foliage, stems, branches or roots, or to soil surrounding the roots, or to the seed before it is planted.

The term "plant" as used herein includes seedlings, bushes and trees. Furthermore, the fungicidal method of the invention includes preventative, protectant, prophylactic and eradicant treatment.

The compounds are preferably used for agricultural and horticultural purposes in the form of a composition. The type of composition used in any instance will depend upon the particular purpose envisaged.

The compositions may be in the form of dusting powders or granules comprising the active ingredient and a solid diluent or carrier, for example fillers such as kaolin, bentonite, keiselguhr, dolomite, calcium carbonate, talc, powdered magnesia, Fuller's earth, gypsum, Hewitt's earth, diatomaceous earth and China clay. Such granules can be preformed granules suitable for application to the soil without further treatment. These granules can be made either by impregnating pellets of filler with the active ingredient or by pelleting a mixture of the active ingredient and powdered filler. Compositions for dressing seed, for example, may comprise an agent (for example a mineral oil) for assisting the adhesion of the composition to the seed; alternatively the active ingredient can be formulated for seed dressing

purposes using an organic solvent (for example N-methylpyrrolidone or dimethylformamide).

The compositions may also be in the form of dispersible powders, granules or grains comprising a wetting agent to facilitate the dispersion in liquids of the powder or grains which may contain also fillers and suspending agents.

The aqueous dispersions or emulsions may be prepared by dissolving the active ingredient(s) in an organic solvent optionally containing wetting, dispersing or emulsifying agent(s) and then adding the mixture to water which may also contain wetting, dispersing or emulsifying agent(s). Suitable organic solvents are ethylene dichloride, isopropyl alcohol, propylene glycol, diacetone alcohol, toluene, kerosene, methylnaphthalene, the xylenes, trichloroethylene, furfuryl alcohol, tetrahydrofurfuryl alcohol, and glycol ethers (e.g. 2-ethoxyethanol and 2-butoxyethanol).

The compositions to be used as sprays may also be in the form of aerosols wherein the formulation is held in a container under pressure in the presence of a propellant, e.g. fluorotrichloromethane or dichlorodifluoromethane.

The compounds can be mixed in the dry state with a pyrotechnic mixture to form a composition suitable for generating in enclosed spaces a smoke containing the compounds.

Alternatively, the compounds may be used in a microencapsulated form.

By including suitable additives, for example additives for improving the distribution, adhesive power and resistance to rain on treated surfaces, the different compositions can be better adapted for various utilities.

The compounds can be used as mixtures with fertilisers (e.g. nitrogen-, potassium- or phosphorus-containing fertilisers). Compositions comprising only granules of fertiliser incorporating, for example coated

with, the compound, are preferred. Such granules suitably contain up to 25% by weight of the compound. The invention therefore also provides a fertiliser composition comprising the compound of general formula (I) or a salt, metal complex, ether or ester; or any isomer of any of the foregoing.

The compositions may also be in the form of liquid preparations for use as dips or sprays which are generally aqueous dispersions or emulsions containing the active ingredient in the presence of one or more surfactants e.g. wetting agent(s), dispersing agent(s), emulsifying agent(s) or suspending agent(s). These agents can be cationic, anionic or non-ionic agents. Suitable cationic agents are quaternary ammonium compounds, for example cetyltrimethylammonium bromide.

Suitable anionic agents are soaps, salts of aliphatic monoesters of sulphuric acid (for example sodium lauryl sulphate), and salts of sulphonated aromatic compounds (for example sodium dodecylbenzenesulphonate, sodium, calcium or ammonium lignosulphonate, butylnaphthalene sulphonate, and a mixture of sodium diisopropyl- and triisopropyl-naphthalene sulphonates).

Suitable non-ionic agents are the condensation products of ethylene oxide with fatty alcohols such as oleyl or cetyl alcohol, or with alkyl phenols such as octyl- or nonyl-phenol and octylcresol. Other non-ionic agents are the partial esters derived from long chain fatty acids and hexitol anhydrides, the condensation products of the said partial esters with ethylene oxide, and the lecithins. Suitable suspending agents are hydrophilic colloids (for example polyvinylpyrrolidone and sodium carboxymethylcellulose), and the vegetable gums (for example gum acacia and gum tragacanth).

The compositions for use as aqueous dispersions or emulsions are generally supplied in the form of a concentrate containing a high proportion of the active ingredient(s), the concentrate to be diluted with water

before use. These concentrates often should be able to withstand storage for prolonged periods and after such storage be capable of dilution with water in order to form aqueous preparations which remain homogeneous for a sufficient time to enable them to be applied by conventional spray equipment. The concentrates may conveniently contain up to 95%, suitably 10-85%, for example 25-60%, by weight of the active ingredient(s). These concentrates suitably contain organic acids (e.g. alkaryl or aryl sulphonic acids such as xylenesulphonic acid or dodecylbenzenesulphonic acid) since the presence of such acids can increase the solubility of the active ingredient(s) in the polar solvents often used in the concentrates. The concentrates suitably contain also a high proportion of surfactants so that sufficiently stable emulsions in water can be obtained. After dilution to form aqueous preparations, such preparations may contain varying amounts of the active ingredient(s) depending upon the intended purpose, but an aqueous preparation containing 0.0005% or 0.01% to 10% by weight of active ingredient(s) may be used.

The compositions of this invention can comprise also other compound(s) having biological activity, e.g. compounds having similar or complementary fungicidal or compounds having herbicidal, plant growth regulating or insecticidal activity.

The other fungicidal compound can be for example one which is capable of combating ear diseases of cereals (e.g. wheat) such as Septoria, Gibberella and Helminthosporium spp., seed and soil borne diseases and downy and powdery mildews on grapes and powdery mildew and scab on apple etc. These mixtures of fungicides can have a broader spectrum of activity than the compound of general formula (I) alone; further the other fungicide can have a synergistic effect on the fungicidal activity of the compound of general formula (I). Examples of the other fungicidal compounds are imazalil, benomyl, carbendazim

(BCM), thiophanate-methyl, captafol, captan, sulphur, dithiocarbamates, carbathiins, copper oxychloride, triforine, dodemorph, tridemorph, dithianon, pyrazophos, binapacryl, quinomethionate, panoctine, furalaxyl, aluminium trio(ethyl phosphonate), DPX3217, ethirimol, dimethirimol, bupirimate, chlorothalonil and metaxanine.

The compound of general formula (I) can be mixed with soil, peat or other rooting media for the protection of plants against seed-borne, soil-borne or foliar fungal diseases.

The following Examples illustrate the invention; the temperatures are given in degrees Centigrade ($^{o}$), and percentages are by weight.

EXAMPLE 1

4-(1,2,4-Triazol-1-yl)-2,2,7,7-tetramethyl-octan-3,5-diol-6-one

Stage 1. Freshly distilled t-butyl glyoxal (0.05 mol) in tetrahydrofuran (THF; 5ml) was added dropwise to a solution of N-methylanilinemagnesium bromide (0.07 mol) in THF (20ml) at 0$^{o}$. Triazole pinacolone (0.05 mol) was added portionwise at 0$^{o}$ and the solution stirred at room temperature for 15 hours. The white precipitate that formed was filtered off to give 4-(1,2,4-triazol-1-yl)-2,2,7,7-tetramethyl-octan-5-ol-3,6-dione as a white crystalline solid, m.p. 170-171$^{o}$.

Stage 2. The product (2.0g) of Stage 1 was dissolved in methanol (70ml) and the solution stirred at 0$^{o}$. Sodium borohydride (0.13g) was added portionwise and the reaction mixture stirred at 0$^{o}$ for 1 hour. The methanol was removed *in vacuo* and the residue treated with water (50ml) and then concentrated hydrochloric acid (2.0ml), and the mixture was extracted with diethyl ether (2 x 50ml). Removal of the ether gave a white foam which was

recrystallised from petroleum ether/chloroform to give, as white needles, the title compound, m.p. 180-182°.

Compound No 2 of Table I was prepared similarly using the appropriate starting substances.

EXAMPLE 2

An emulsifiable concentrate was made up by mixing the ingredients, and stirring the mixture until all the constituents were dissolved.

| | |
|---|---|
| Compound No 1 of Table I | 10% |
| Ethylene dichloride | 40% |
| Calcium dodecylbenzenesulphate | 5% |
| "Lubrol" L | 10% |
| "Aromasol" H | 35% |

EXAMPLE 3

A composition in the form of grains readily dispersible in a liquid, e.g. water, was prepared by grinding together the first three ingredients in the presence of added water and then mixing in the sodium acetate. The resultant mixture was dried and passed through a British Standard mesh sieve, size 44-100, to obtain the desired size of grains.

| | |
|---|---|
| Compound No 2 of Table I | 50% |
| "Dispersol" T | 25% |
| "Lubrol" APN5 | 1.5% |
| Sodium acetate | 23.5% |

EXAMPLE 4

The ingredients were all ground together to produce a powder formulation readily dispersible in liquids.

| | |
|---|---|
| Compound No 1 of Table I | 45% |
| "Dispersol" T | 5% |
| "Lissapol" NX | 0.5% |
| "Cellofas" B600 | 2% |
| Sodium acetate | 47.5% |

EXAMPLE 5

The active ingredient was dissolved in a solvent and the resultant liquid was sprayed on to the granules of China clay. The solvent was then allowed to evaporate to produce a granular composition.

| | |
|---|---|
| Compound No 2 of Table I | 5% |
| China clay granules | 95% |

EXAMPLE 6

A composition suitable for use as a seed dressing was prepared by mixing the three ingredients.

| | |
|---|---|
| Compound No 1 of Table I | 50% |
| Mineral oil | 2% |
| China clay | 48% |

EXAMPLE 7

A dusting powder was prepared by mixing the active ingredient with talc.

| | |
|---|---|
| Compound No 2 of Table I | 5% |
| Talc | 95% |

EXAMPLE 8

A Col formulation was prepared by ball-milling the constituents set out below and then forming an aqueous suspension of the ground mixture with water.

| | |
|---|---|
| Compound No 1 of Table I | 40% |
| "Dispersol" T | 10% |
| "Lubrol" APN5 | 1% |
| Water | |

EXAMPLE 9

A dispersible powder formulation was made by mixing together the ingredients set out below and then grinding the mixture until all were thoroughly mixed.

| | |
|---|---|
| Compound No 2 of Table I | 25% |
| "Aerosol" OT/B | 2% |
| "Dispersol" AC | 5% |
| China clay | 28% |
| Silica | 40% |

EXAMPLE 10

This Example illustrates the preparation of a dispersible powder formulation. The ingredients were mixed and the mixture then ground in a comminution mill.

| | |
|---|---|
| Compound No 1 of Table I | 25% |
| "Perminal" BX | 1% |
| "Dispersol" T | 5% |
| Polyvinylpyrrolidone | 10% |
| Silica | 25% |
| China clay | 34% |

EXAMPLE 11

The ingredients set out below were formulated into a dispersible powder by mixing then grinding the ingredients.

| | |
|---|---|
| Compound No 2 of Table I | 25% |
| "Aerosol" OT/B | 2% |
| "Dispersol" A | 5% |
| China clay | 68% |

In Examples 2 to 11 the proportions of the ingredients given are by weight.

There now follows an explanation of the compositions or substances represented by the various Trade Marks and Trade Names mentioned above.

| | |
|---|---|
| LUBROL L: | a condensate of nonyl phenol (1 mole) with ethylene oxide (13 moles) |
| AROMASOL H: | a solvent mixture of alkylbenzenes |
| DISPERSOL T & AC: | a mixture of sodium sulphate and a condensate of formaldehyde with sodium naphthalene sulphonate |

| | |
|---|---|
| LUBROL APN5: | a condensate of nonyl phenol (1 mole) with naphthalene oxide (5.5 moles) |
| CELLOFAS B600: | a sodium carboxymethyl cellulose thickener |
| LISSAPOL NX: | a condensate of nonyl phenol (1 mole) with ethylene oxide (8 moles) |
| AEROSOL OT/B: | dioctyl sodium sulphosuccinate |
| PERMINAL BX: | a sodium alkyl naphthalene sulphonate |

EXAMPLE 12

The compounds were tested against a variety of foliar fungal diseases of plants. The techniques employed were as follows:

The plants were grown in John Innes Potting Compost (No. 1 or 2) in 4cm diameter mini-pots. A layer of fine sand was placed at the bottom of the pots containing the dicotyledonous plants to facilitate the uptake of test compound by the roots. The test compounds were formulated either by bead-milling with aqueous 'Dispersol' T or as a solution in acetone or acetone/ethanol which was diluted to the required concentration immediately before use. For the foliage diseases, suspensions (100 ppm active ingredient) were sprayed on to the foliage and applied to the roots of the same plant via the soil. Exceptions were the tests on Botrytis cinerea and Venturia inaequalis, in which the compound was sprayed on to the foliage only. Sprays were applied to maximum retention and root drenches to a final concentration equivalent to approximately

40 ppm a.i./dry soil. Tween 20, to give a final concentration of 0.05% was added when the sprays were applied to cereals.

For most of the tests the compound was applied to the soil (roots) and to the foliage (by spraying) one or two days before the plant was inoculated with the diseases. An exception was the test on Erysiphe graminis in which the plants were inoculated 24 hours before treatment. After inoculation, the plants were put into an appropriate environment to allow infection to take place and then incubated until the disease was ready for assessment. The period between inoculation and assessment varied from four to fourteen days according to the disease and environment.

The disease control was recorded by the following grading:

4 = No disease
3 = 0-5% "
2 = 6-25% "
1 = 26-60% "
0 = $>$ 60% "

The results are shown in Table II.

TABLE II

| COMPOUND NO | PUCCINIA RECONDITA (wheat) | PHYTOPHTHORA INFESTANS (tomato) | BOTRYTIS CINEREA (tomato) | ERYSIPHE GRAMINIS (barley) | CERCOSPORA ARACHIDICOLA (peanut) | VENTURIA INAEQUALIS (apple) |
|---|---|---|---|---|---|---|
| 1 | 4 | 1 | 1 | 4 | - | 1 |
| 2 | 3 | - | 0 | 4 | 2 | 1 |

"-" signifies compound not tested

0007707



1. Triazole and imidazole compounds having the general formula (I):

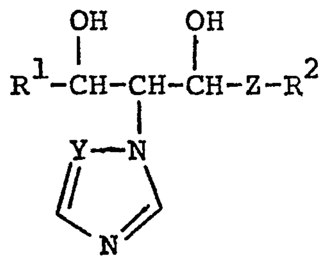

(I)

wherein each of $R^1$ and $R^2$, which may be the same or different, is unsubstituted or alkyl-substituted cycloalkyl, unsubstituted or halo-substituted alkyl or unsubstituted or substituted phenyl, Y is =N- or =CH- and Z is C=O or a functional derivative thereof such as an imine, oxime, ketal , hydrazone or semicarbazone; or an ester, ether, acid addition salt or metal complex thereof; and isomers of any of the foregoing.

2. Compounds as claimed in claim 1 wherein each of $R^1$ and $R^2$, which may be the same or different, is a straight or branched chain alkyl group containing from 1 to 5 carbon atoms optionally substituted with 1 to 3 halogen atoms; or a cycloalkyl group having 3 to 6 carbon atom; or a phenyl group optionally substituted with one or more halogen atoms or $C_{1-4}$ alkyl [e.g. methyl, ethyl, propyl (n- or i-propyl) and butyl (n-, i- or t-butyl)], halo($C_{1-4}$ alkyl) (e.g. chloro- or bromo- methyl), hydroxy ($C_{1-4}$ alkyl) (e.g. hydroxymethyl), phenyl, halophenyl (e.g. chlorophenyl), cycloalkyl, nitro, cyano, $C_{1-4}$ alkoxy (e.g. methoxy, ethoxy, propoxy or butoxy), $C_{2-4}$ alkenyloxy (e.g. allyloxy), ($C_{1-4}$ alkylene)-dioxy (e.g. methylenedioxy), ($C_{1-4}$ alkoxy) ($C_{1-4}$

alkyl) [e.g. methoxy- or ethoxy- methyl or 2-methoxy- or ethoxy-ethyl], mercapto, ($C_{1-4}$ alkyl) thio [e.g. methyl- or ethyl-thio], ($C_{1-4}$ alkyl) sulphonyl [e.g. methyl- or ethyl-sulphonyl], ($C_{1-4}$ haloalkyl)sulphonyl [e.g. trifluoromethylsulphonyl], phenylsulphonyl, unsubstituted or mono- or di-($C_{1-4}$ alkyl) substituted sulphamoyl or carbamoyl, 1-pyrrolidinylsulphonyl, carboxy, ($C_{1-4}$ alkoxy)-carbonyl [e.g. methoxy- or ethoxy-carbonyl], hydroxy, $C_{1-6}$ alkanoyloxy, benzoyloxy, carboxy ($C_{1-4}$ alkyl)oxy (e.g. carboxymethoxy or 1-carboxyethoxy), unsubstituted or mono- or di- ($C_{1-4}$ alkyl) substituted amino, ($C_{1-6}$ alkanoyl)amino, N-($C_{1-4}$ alkyl)-N-($C_{1-6}$ alkanoyl)amino, formylamino, N-($C_{1-4}$ alkyl) formylamino, phenylethyl, methylenedioxyphenyl, phenoxy or benzyloxy groups.

3. The specific compounds exemplified in the preceding descriptive passages and, in particular, the specific compounds set out in Table I.

4. A process for preparing the compounds claimed in claim 1, 2 or 3 which comprises selectively reducing a diketo-alcohol of general formula (II):

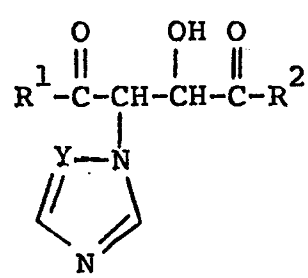

(II)

wherein Y, $R^1$ and $R^2$ are as defined, or a salt or metal complex thereof, with a reducing agent such as a metal hydride reducing agent (e.g. lithium aluminium hydride, sodium borohydride or aluminium isopropoxide) in an inert polar solvent (e.g. water or ethanol).

5. A process for preparing the diketo-alcohols of general formula (II) in claim 4 which comprises reacting a compound of general formula (III):

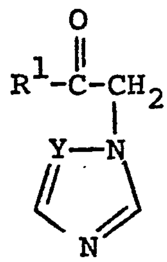

(III)

where Y and $R^1$ are as defined, with a compound of general formula (IV):

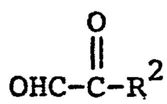

(IV)

where $R^2$ is as defined, in the presence of a base (e.g. sodium methoxide or N-methylaniline magnesium bromide).

6. A composition for combating fungi or regulating plant growth comprising, as an active ingredient, a compound as claimed in claim 1, 2 or 3, or a functional derivative, ester, ether, salt or metal complex of such a compound, or an isomer of any of the foregoing; together with a carrier or diluent for the active ingredient.

7. A method of combating fungal diseases or regulating plant growth which comprises applying to a plant, or to seed, or to the locus of a plant or seed, a compound as claimed in claim 1, 2 or 3, or a composition as claimed in claim 6, or a functional derivative, ester, ether, salt or metal complex of such a compound, or an isomer of any of the foregoing.

0007707

European Patent Office

# EUROPEAN SEARCH REPORT

Application number
EP 79301247.7

| DOCUMENTS CONSIDERED TO BE RELEVANT | | | CLASSIFICATION OF THE APPLICATION (Int. Cl.3) |
|---|---|---|---|
| Category | Citation of document with indication, where appropriate, of relevant passages | Relevant to claim | |
| A | CH - A - 515 252 (ROHM)<br>+ Column 1 - 6 +<br>-- | 1 - 5 | C 07 D 249/08<br>C 07 D 233/64<br>A 01 N 43/50<br>A 01 N 43/64 |
| A | US - A - 4 053 616 (BÜCHEL)<br>+ Abstract +<br>-- | 6,7 | |
| A | US - A - 3 952 002 (KRAMER)<br>+ Abstract +<br>-- | 1 - 5 | |
| A | DE - A - 2 552 967 (BAYER)<br>+ Claims 1 - 5 +<br>---- | 6,7 | TECHNICAL FIELDS SEARCHED (Int.Cl. 3)<br>C 07 C 249/08<br>C 07 D 233/64<br>A 01 N 43/50<br>A 01 N 43/64 |

CATEGORY OF CITED DOCUMENTS
X: particularly relevant
A: technological background
O: non-written disclosure
P: intermediate document
T: theory or principle underlying the invention
E: conflicting application
D: document cited in the application
L: citation for other reasons

&: member of the same patent family, corresponding document

X The present search report has been drawn up for all claims

| Place of search | Date of completion of the search | Examiner |
|---|---|---|
| VIENNA | 27-09-1979 | HAMMER |